# EUROPÄISCHE PATENTANMELDUNG

(11) **EP 2 085 101 A2**
(43) Veröffentlichungstag der Anmeldung: **05.08.2009**
(21) Anmeldenummer: 08171336.4
(22) Anmeldetag: 11.12.2008
(51) Int. Cl.: A61L 31/10, A61L 31/02, A61L 31/14

(54) **Implantat mit einem Grundkörper aus einer biokorrodierbaren Legierung**

(30) Priorität: 30.01.2008 DE 102008006654
(71) Anmelder: Biotronik VI Patent AG, 6341 Baar (CH)
(72) Erfinder: Adden, Nina, 90427, Nürnberg (DE)
(74) Vertreter: Lindner-Vogt, Karin L.

(57) **Zusammenfassung**

Die Erfindung betrifft ein Implantat mit einem Grundkörper, der ganz oder in Teilen aus einem biokorrodierbaren, metallischen Werkstoff besteht, und bei dem zumindest die aus dem biokorrodierbaren, metallischen Werkstoff bestehenden Teile des Grundkörpers vollständig oder teilweise mit einer Beschichtung bedeckt sind, die ihrerseits aus einem Polymer besteht oder ein Polymer enthält. Erfindungsgemäß besteht das Polymer zu mindestens 90% der Gesamtzahl der vorhandenen Polymereinheiten aus Polymereinheiten der Formel (1) und Polymereinheiten der Formel (2) 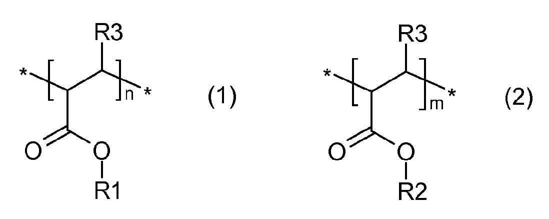 wobei R1 Alkyl, Hydroxyalkyl, Alkoxyalkyl oder Cycloalkyl ist; R2 ein in künstlichem Plasma hydrolisierbarer Silylrest ist; R3 Wasserstoff oder Methyl ist; und n + m = 10 bis 20.000 ist, mit der Maßgabe, dass ein Verhältnis von n zu m im Bereich von 1 : 9 bis 9 : 1 liegt.

## Beschreibung

Die Erfindung betrifft ein Implantat mit einem Grundkörper, der ganz oder in Teilen aus einem biokorrodierbaren, metallischen Werkstoff besteht, und bei dem zumindest die aus dem biokorrodierbaren, metallischen Werkstoff bestehenden Teile des Grundkörpers vollständig oder teilweise mit einer Beschichtung bedeckt sind, die ihrerseits aus einem Polymer besteht oder ein Polymer enthält.

### Technologischer Hintergrund und Stand der Technik

Implantate haben in vielfältiger Ausführungsform Anwendung in der modernen Medizintechnik gefunden. Sie dienen unter anderem der Unterstützung von Gefäßen, Hohlorganen und Gangsystemen (endovaskuläre Implantate), zur Befestigung und temporären Fixierung von Gewebeimplantaten und Gewebstransplantationen, aber auch zu orthopädischen Zwecken, zum Beispiel als Nagel, Platte oder Schraube.

So hat sich beispielsweise die Implantation von Stents als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Der Grundkörper jedes Implantats, insbesondere von Stents, besteht aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems, in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden. Für die Zwecke der vorliegenden Erfindung sind lediglich degradierbare/resorbierbare, metallische Implantatwerkstoffe von Interesse, die nachfolgend als biokorrodierbare metallische Werkstoffe bezeichnet werden.

Der Einsatz biokorrodierbarer metallischer Werkstoffe bietet sich insbesondere schon deshalb an, da zumeist nur ein zeitweiliger Verbleib des Implantats im Körper zur Erfüllung des medizinischen Zweckes erforderlich ist. Implantate aus permanenten Werkstoffen, also Werkstoffen, die im Körper nicht abgebaut werden, sind wieder zu entfernen, da es mittel- und langfristig auch bei hoher Biokompatibilität zu Abstoßungsreaktionen des Körpers kommen kann.

Ein Ansatz zur Vermeidung eines weiteren chirurgischen Eingriffs besteht demnach darin, das Implantat ganz oder in Teilen aus einem biokorrodierbaren metallischen Werkstoff zu formen. Unter Biokorrosion werden mikrobielle Vorgänge oder schlicht durch die Anwesenheit von Körpermedien bedingte Prozesse verstanden, die zu einem allmählichen Abbau der aus dem Werkstoff bestehen Struktur führen. Zu einem bestimmten Zeitpunkt verliert das Implantat oder zumindest der Teil des Implantates, der aus dem biokorrodierbaren Werkstoff besteht, seine mechanische Integrität. Die Abbauprodukte werden vom Körper weitgehend resorbiert. Die Abbauprodukte haben, wie beispielsweise beim Magnesium, im günstigsten Fall sogar eine positive therapeutische Wirkung auf das umgebende Gewebe. Geringe Mengen nicht resorbierbarer Legierungsbestandteile sind tolerierbar.

Bekannte biokorrodierbare metallische Werkstoffe umfassen Reineisen und biokorrodierbare Legierungen der Hauptelemente Magnesium, Eisen, Zink, Molybdän und Wolfram. In DE 197 31 021 A1 wird unter anderem vorgeschlagen, medizinische Implantate aus einem metallischen Werkstoff zu formen, dessen Hauptbestandteil ein Element aus der Gruppe Alkalimetalle, Erdalkalimetalle, Eisen, Zink und Aluminium ist. Als besonders geeignet werden Legierungen auf Basis von Magnesium, Eisen und Zink beschrieben. Nebenbestandteile der Legierungen können Mangan, Cobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90%, Yttrium 3,7 - 5,5%, Seltenerdmetallen 1,5 - 4,4% und Rest <1% bekannt, die sich insbesondere zur Herstellung einer Endoprothese, z. B. in Form eines Stents, eignet. Ungeachtet der erreichten Fortschritte auf dem Gebiet biokorrodierbarer Metalllegierungen sind die bisher bekannten Legierungen aufgrund ihres Korrosionsverhaltens nur beschränkt einsatzfähig. Insbesondere limitiert die relativ rasche Biokorrosion der Magnesiumlegierungen deren Einsatzgebiet.

Herkömmliche technische Einsatzgebiete von Formkörpern aus metallischen Werkstoffen, insbesondere Magnesiumlegierungen, außerhalb der Medizintechnik erfordern in der Regel eine weitgehende Unterbindung korrosiver Prozesse. Dementsprechend ist die Zielstellung der meisten technischen Verfahren zur Verbesserung des Korrosionsverhaltens eine vollständige Inhibierung korrosiver Prozesse. Dagegen sollte die Zielstellung zur Verbesserung des Korrosionsverhaltens der hier vorliegenden biokorrodierbaren metallischen Werkstoffe nicht in der vollständigen Unterbindung, sondern nur in der Hemmung korrosiver Prozesse liegen. Schon aus diesem Grunde eignen sich die meisten bekannten Maßnahmen zur Verbesserung des Korrosionsschutzes nicht. Ferner müssen für einen medizin-technischen Einsatz auch toxikologische Aspekte berücksichtigt werden. Des Weiteren sind korrosive Prozesse stark von dem Medium abhängig, in dem sie ablaufen, und daher dürfte eine Übertragbarkeit der unter herkömmlichen Umweltbedingungen auf technischem Gebiet gewonnenen Erkenntnisse zum Korrosionsschutz auf die Prozesse in physiologischer Umgebung nicht uneingeschränkt möglich sein.

Ein Ansatzpunkt bekannter technischer Verfahren zur Verbesserung des Korrosionsverhaltens (im Sinne einer Verstärkung des Korrosionsschutzes) sieht vor, eine korrosionsschützende Schicht auf dem aus dem metallischen Werkstoff bestehenden Formkörper zu erzeugen. Bekannte Verfahren zur Erzeugung einer korrosionsschützenden Schicht wurden unter dem Gesichtspunkt eines technischen Einsatzes des beschichteten Formkörpers - jedoch nicht medizin-technischen Einsatzes in biokorrodierbaren Implantaten in physiologischer Umgebung - entwickelt und optimiert. Diese bekannten Verfahren umfassen beispielsweise: das Aufbringen von Polymeren oder anorganischen Deckschichten, das Erzeugen einer Emaille, die chemische Konversion der Oberfläche, Heißgasoxidation, Anodisieren, Plasmaspritzen, Laserstrahl-Umschmelzen, PVD-Verfahren, lonen-Implantation oder Lackieren.

Der vorliegenden Erfindung liegt die Aufgabe zugrunde, eine verbesserte oder zumindest alternative Beschichtung für ein Implantat aus einem biokorrodierbaren metallischen Werkstoff bereitzustellen, die eine temporäre Inhibition, aber nicht vollständige Unterbindung der Korrosion des Werkstoffs in physiologischer Umgebung bewirkt.

### Zusammenfassung der Erfindung

Die Erfindung geht aus von einem Implantat mit einem Grundkörper, der ganz oder in Teilen aus einem biokorrodierbaren, metallischen Werkstoff besteht, und bei dem zumindest die aus dem biokorrodierbaren, metallischen Werkstoff bestehenden Teile des Grundkörpers vollständig oder teilweise mit einer Beschichtung bedeckt sind, die ihrerseits aus einem Polymer besteht oder ein Polymer enthält. Erfindungsgemäß besteht das Polymer zu mindestens 90% der Gesamtzahl der vorhandenen Polymereinheiten aus Polymereinheiten der Formel (1) und Polymereinheiten der Formel (2) 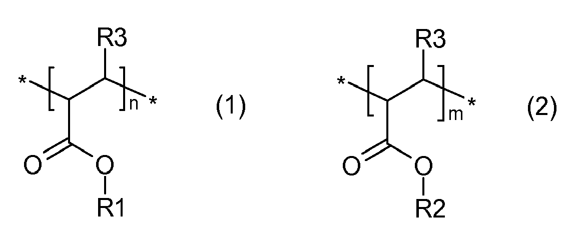 wobei R1 Alkyl, Hydroxyalkyl, Alkoxyalkyl oder Cycloalkyl ist; R2 ein in künstlichem Plasma hydrolisierbarer Silylrest ist; R3 Wasserstoff oder Methyl ist; und n + m = 10 bis 20.000 ist, bevorzugt 300 bis 3.000, mit der Maßgabe, dass ein Verhältnis von n zu m in Bereich von 1:9 bis 9:1 liegt.

Der Erfindung liegt die Erkenntnis zugrunde, dass Polymere auf Basis der genannten Ester der Acrylsäure/Methacrylsäure ein überwiegend hydrophobes Verhalten zeigen und dementsprechend in wässrigen Medien, wie insbesondere künstlichem Plasma, allenfalls eine geringfügige Löslichkeit besitzen. Werden diese Polymere demnach als Beschichtungsmaterial für zumindest die Teile eines Implantats benutzt, die aus einem biokorrodierbaren, metallischen Werkstoff bestehen, so ist dieser Werkstoff zunächst durch die Beschichtung vor korrosivem Abbau geschützt. Allmählich erfolgt im künstlichen Medium jedoch eine Hydrolyse der Silylester der Polymereinheiten der Formel (2). Damit einhergehend verstärkt sich der hydrophile Charakter des Polymers und seine Löslichkeit in künstlichem Plasma erhöht sich. Mit anderen Worten, mit fortschreitender Hydrolyse der Polymereinheiten der Formel (2) erhöht sich die Löslichkeit des Polymers und sobald der Abbau hinreichend fortgeschritten ist, wird der darunter liegende biokorrodierbare, metallische Werkstoff abgebaut.

Ein wesentlicher Unterschied im Vergleich zu Beschichtungen aus bekannten biokorrodierbaren Polymeren liegt darin, dass durch die geschilderte Hydrolyse der Silylester nicht die Monomere als Abbauprodukt erhalten werden, sondern vielmehr das kettenförmige Grundgerüst des Polymers erhalten bleibt. Dies hat den Vorteil, dass eine hohe Belastung des Gewebes mit niedermolekularen Abbauprodukten im Vergleich zu herkömmlichen, biokorrodierbaren Polymeren vermieden werden kann. Gerade diese niedermolekularen Abbauprodukte stehen in Verdacht, Ausgangspunkt unerwünschter Reaktionen im Körper des Patienten zu sein, die den Heilungsverlauf negativ beeinflussen können oder schlimmstenfalls gar unterbinden. Die durch die Hydrolyse der Silylester erhaltenen löslichen Polymere werden dagegen weitgehend ohne negative Wechselwirkungen ausgeschieden.

Das Polymer enthält die Polymereinheiten der Formeln (1) und (2), ist also ein Copolymer, das mindestens jeweils eine Spezies als Polymereinheit enthält, die unter besagte Formeln (1) und (2) fällt. Demnach ist auch denkbar, dass das Polymer verschiedene, jeweils unter die Polymereinheiten der Formeln (1) oder (2) zu subsumierende Spezies beinhaltet. Eine Verteilung der Polymereinheiten in dem Polymer ist für die Zwecke der Erfindung von untergeordneter Bedeutung, so dass in der Regel auch Block- oder Pfropfpolymere Anwendung finden können. Der Begriff Copolymer ist eine allgemeine Bezeichnung für Polymere, die aus zwei oder mehreren verschiedenen Arten von Monomeren bestehen und durch gemeinsame Copolymerisation hergestellt werden. Das Polymer kann ferner bis zu 10% Polymereinheiten aufweisen, die durch radikalische Polymerisation eines von den Formeln (1) und (2) abweichenden Vinylmonomers entstehen; zum Beispiel kann diese weitere Polymereinheit Vinylpyrrolidon oder Vinylacetat sein. Hierdurch kann zum Beispiel das Löslichkeitsverhalten des Polymers zusätzlich beeinflusst werden.

Als Prüfmedium der Hydrolisierbarkeit der Silylester beziehungsweise des Löslichkeitsverhaltens der Polymere dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/I). Eine Probe des zu untersuchenden Polymers wird dazu in einem verschlossenen Probenbehälter mit einer definierten Menge des Prüfmediums bei 37°C gelagert. In zeitlichen Abständen - abgestimmt auf das zu erwartende Abbauverhalten des Silylesters - von wenigen Stunden bis zu mehreren Monaten wird an den Proben oder durch Untersuchung des künstlichen Mediums in bekannter Weise ein Umfang des hydrolytischen Abbaus bestimmt. Das künstliche Plasma nach EN ISO 10993-15:2000 entspricht einem blutähnlichen Medium und stellt damit eine Möglichkeit dar, eine physiologische Umgebung reproduzierbar nachzustellen.

Nach einer bevorzugten Ausführungsform weist das Polymer durch Vorgabe der Substituenten R1 bis R3 sowie Festlegung von n und m eine Löslichkeit in künstlichem Plasma bei einer Temperatur von 37°C auf, die nach 24 Stunden in diesem Plasma geringer ist als 0.01 g/l. Ergänzend oder alternativ hierzu weist das Polymer durch Vorgabe der Substituenten R1 bis R3 sowie Festlegung von n und m eine Löslichkeit in künstlichem Plasma bei einer Temperatur von 37°C auf, die nach 30 Tagen in diesem Plasma im Bereich von 0.2 bis 0.5 g/l liegt. Die vorgenannten Vorgaben für die Löslichkeit nach 24 Stunden bzw. 30 Tagen sollen sicherstellen, dass der Abbau der unter der polymeren Beschichtung liegenden biodegradierbaren, metallischen Bestandteile des Implantats nicht sofort nach Implantation einsetzt, sondern gehemmt wird. Nach 30 Tagen soll jedoch ein merklicher Abtransport des Polymers in Folge der sich einstellenden Erhöhung der Löslichkeit stattfinden. Letzterer Zeitraum orientiert sich an dem Umstand, dass für viele Implantate eine Funktionalität der Bestandteile aus dem biodegradierbaren, metallischen Werkstoff nur für zwei bis drei Monate aufrechterhalten werden muss. Danach sollte dieser Bestandteil des Implantats zügig abgebaut werden. Die Löslichkeit des Polymers nach 30 Tagen muss daher hinreichend hoch sein, um den genannten Zeitrahmen einzuhalten. Zur Erzielung des angegebenen Löslichkeitsverhaltens kann der Fachmann wie folgt vorgehen:
Ausgehend von einem konkreten Polymer, z. B. erhältlich durch Polymerisation von Methylmethacrylat und Trimethylsilylmethacrylat, wird eine Löslichkeit dieses Polymers nach 24 Stunden bzw. 30 Tagen bei einer Temperatur von 37°C in künstlichem Plasma bestimmt. Besitzt die Löslichkeit nicht die für die spezifische Applikation gewünschten Eigenschaften, so kann der Fachmann über folgende Variationen Einfluss nehmen:
   (1) Anstelle von Methyl als Rest R1 der Polymereinheit der Formel (1) wird ein langkettigerer Alkylrest oder ein Cycloalkylrest gewählt, sofern die Hydrophobität des polymeren Materials gesteigert werden soll. Umgekehrt wird ein hydrophilerer Rest, also ein Hydroxyalkylrest oder ein kurzkettiger Alkoxyalkylrest gewählt, wenn die Hydrophilie und damit die Löslichkeit gesteigert werden soll.
   (2) Eine Hydrolysegeschwindigkeit der Silylester nimmt in der Regel mit zunehmender Größe der Substituenten am Silicium ab. Demnach kann durch Wahl eines Silylrestes, der relativ langsam hydrolisiert wird, das Erreichen der notwendigen Löslichkeit zum Abtransport der Polymere zeitlich hinausgezögert werden.
   (3) Die Löslichkeit des Polymers in künstlichem Plasma hängt natürlich auch von der Anzahl der hydrophilen Gruppen ab, die vornehmlich durch Hydrolyse der Silylester entstehen. Erhöht der Fachmann demnach den Anteil der Silylester, also der Polymereinheiten der Formel (2) am Polymer, so wird das vollständig hydrolisierte Polymer eine deutlich erhöhte Löslichkeit gegenüber einem Polymer mit weniger Silylresten aufweisen.
   (4) Weiterhin ist die Löslichkeit auch in einem geringeren Maße vom Molekulargewicht des Polymers abhängig. In der Regel geht mit einer Erhöhung des Molekulargewichtes eine Verschlechterung der Löslichkeit im künstlichen Plasma einher.
Der Fachmann hat demnach vier Parameter, deren Einfluss er tendenziell kennt und die er unabhängig voneinander variieren kann, um zu einem Produkt mit den für die spezifische Applikation gewünschten Eigenschaften zu gelangen.
Vorzugsweise ist R1 ein unsubstituierter, hydroxy- oder C1-C10-alkoxysubstituierter C1-C10-Alkylrest oder ein C2-C10-Cycloalkylrest.
Besonders bevorzugt ist R1 Methyl, Ethyl, Propyl, 2-Methylethyl, Butyl, 2-Methylpropyl, 2,3-Dimethylethyl, Cyclohexyl, 2-Hydroxyethyl, 3-Hydroxypropyl oder 2-Methoxyethyl. Ganz besonders bevorzugt ist R1 Methyl, Ethyl, Propyl, oder Butyl. Insbesondere ist R1 Methyl.
Weiterhin ist bevorzugt, insbesondere auch mit den zuvor genannten Ausführungsformen für den Substituenten R1, dass R2 Trimethylsilyl, Triethylsilyl, Tripropylsilyl oder Tributylsilyl ist. Insbesondere ist R2 Trimethylsilyl oder Triethylsilyl.
Ferner ist bevorzugt, wenn ein Verhältnis von n zu m im Bereich von 1:2,33 bis 1,5:1 liegt.
Besonders bevorzugt ist ferner eine Ausführungsform, bei der die Polymereinheit der Formel (1) Methylmethacrylat oder Methylacrylat und die Polymereinheit der Formel (2) Trimethylsilylmethacrylat ist.
Bevorzugte Varianten sehen ferner vor, dass ein mittleres Molekulargewicht der Polymere im Bereich von 1.000 bis 1.000.000 g/mol, besonders bevorzugt im Bereich von 5.000 bis 500.000 g/mol, insbesondere 40.000 bis 200.000 g/mol, liegt.
Der polymeren Beschichtung können Additive zugesetzt werden, zum Beispiel um die Verarbeitung zu erleichtern. Denkbar ist auch, dass die Beschichtung als Matrix für Wirkstoffe dient, die nach der Implantation in die Gewebsumgebung freigesetzt werden.
Die Polymere können in bekannter Weise durch freie radikalische Polymerisation hergestellt werden, z. B. in Analogie zu der Vorschrift von P. Durand et al. (POLYMER 1994, Vol. 35, Seiten 4392 bis 4396). Das Molekulargewicht kann unter anderem über die Konzentration des Initiators für die radikalische Polymerisation beeinflusst werden.
Vorzugsweise ist der biokorrodierbare metallische Werkstoff Reineisen oder eine biokorrodierbare Legierung ausgewählt aus der Gruppe der Elemente Magnesium, Eisen, Zink, Molybdän und Wolfram; insbesondere ist der Werkstoff eine biokorrodierbare Magnesiumlegierung. Unter Legierung wird vorliegend ein metallisches Gefüge verstanden, deren Hauptkomponente Magnesium, Eisen, Zink, Molybdän oder Wolfram ist. Hauptkomponente ist die Legierungskomponente, deren Gewichtsanteil an der Legierung am höchsten ist. Ein Anteil der Hauptkomponente beträgt vorzugsweise mehr 50 Gew.%, insbesondere mehr als 70 Gew.%.

Besonders bevorzugt ist eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt. Diese Magnesiumlegierung bestätigte bereits in klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden. Bevorzugt sind weiterhin Magnesiumlegierungen die bis zu 6 Gew.% Zink enthalten. Besonders bevorzugt ist weiterhin eine Magnesiumlegierung der Zusammensetzung Yttrium 0,5 - 10 Gew.%, Zink 0,5 - 6 Gew.%, Calcium 0,05 - 1 Gew.%, Mangan 0 - 0,5 Gew.%, Silber 0 - 1 Gew.%, Cer 0 - 1 Gew.% sowie Zirkonium 0 - 1 Gew.% oder Silizium 0 - 0,4 Gew.%, wobei sich die Angaben auf Gew.% an der Legierung beziehen und Magnesium sowie herstellungsbedingte Verunreinigungen den auf 100 Gew.% verbleibenden Restanteil an der Legierung einnehmen.

Die Legierungen der Elemente Magnesium, Eisen, Zink, Molybdän oder Wolfram sind so in ihrer Zusammensetzung zu wählen, dass sie biokorrodierbar sind. Als biokorrodierbar im Sinne der Erfindung werden Legierungen bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist. Die Legierung ist demnach so in ihrer Zusammensetzung zu wählen, dass sie biokorrodierbar ist.

Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/I). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung reproduzierbar nachzustellen.

Der Begriff Korrosion bezieht sich vorliegend auf die Reaktion eines metallischen Werkstoffes mit seiner Umgebung, wobei eine messbare Veränderung des Werkstoffs bewirkt wird, die - bei Einsatz des Werkstoffs in einem Bauteil - zu einer Beeinträchtigung der Funktion des Bauteils führt. Ein Korrosionssystem besteht vorliegend aus dem korrodierenden metallischen Werkstoff sowie einem flüssigen Korrosionsmedium, das in seiner Zusammensetzung die Verhältnisse in physiologischer Umgebung nachstellt oder ein physiologisches Medium, insbesondere Blut ist. Werkstoffseitig beeinflussen die Korrosion Faktoren, wie die Zusammensetzung und Vorbehandlung der Legierung, mikro- und submikroskopische Inhomogenitäten, Randzoneneigenschaften, Temperatur- und mechanischer Spannungszustand und insbesondere die Zusammensetzung einer die Oberfläche bedeckenden Schicht. Auf Seiten des Mediums wird der Korrosionsprozess durch Leitfähigkeit, Temperatur, Temperaturgradienten, Azidität, Volumen-OberflächenVerhältnis, Konzentrationsunterschied sowie Strömungsgeschwindigkeit beeinflusst.

An der Phasengrenzfläche zwischen Werkstoff und Medium laufen Redox-Reaktionen ab. Für eine schützende bzw. hemmende Wirkung müssen vorhandene Schutzschichten und/oder die Produkte der Redox-Reaktionen eine gegen das Korrosionsmedium ausreichend dichte Struktur ausbilden, eine bezogen auf die Umgebung erhöhte thermodynamische Stabilität aufweisen und im Korrosionsmedium wenig löslich oder unlöslich sein. In der Phasengrenzfläche, genauer in einer sich in diesem Bereich ausbildenden Doppelschicht, laufen Ad- und Desorptionsprozesse ab. Die Vorgänge in der Doppelschicht sind geprägt von den dort ablaufenden kathodischen, anodischen und chemischen Teilprozessen. Fremdstoffablagerungen, Verunreinigungen und Korrosionsprodukte beeinflussen den Korrosionsprozess. Die Vorgänge bei der Korrosion sind demnach hoch komplex und lassen sich gerade im Zusammenhang mit einem physiologischen Korrosionsmedium, also Blut oder künstlichem Plasma, nicht oder nur im geringen Umfang voraussagen, da Vergleichsdaten fehlen. Schon aus diesem Grunde ist das Auffinden einer korrosionshemmenden Beschichtung, d. h. einer Beschichtung, die nur zur temporären Herabsetzung der Korrosionsrate eines metallischen Werkstoffs der weiter oben genannten Zusammensetzung in physiologischer Umgebung dient, eine außerhalb der Routine eines Fachmanns liegende Maßnahme.

Der Vorgang der Korrosion lässt sich durch Angabe einer Korrosionsrate quantifizieren. Ein zügiger Abbau ist mit einer hohen Korrosionsrate verbunden, und umgekehrt. Bezogen auf den Abbau des gesamten Formkörpers wird eine im Sinne der Erfindung modifizierte Oberfläche zur Herabsetzung der Korrosionsrate führen. Im Fall von Koronarstents sollte vorzugsweise die mechanische Integrität der Struktur über einen Zeitraum von drei Monaten nach Implantation aufrechterhalten werden.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren. Das Implantat besteht ganz oder in Teilen aus dem biokorrodierbaren Werkstoff. Wenn das Implantat nur in Teilen aus dem biokorrodierbaren Werkstoff besteht, so ist dieser Teil entsprechend zu beschichten.

Vorzugsweise ist das Implantat ein Stent. Stents herkömmlicher Bauart weisen eine filigrane Struktur aus metallischen Streben auf, die zur Einbringung in den Körper zunächst in einem nicht-expandierten Zustand vorliegt und die am Ort der Applikation dann in einen expandierten Zustand aufgeweitet wird. Bei Stents bestehen besondere Anforderungen an die korrosionshemmende Schicht: Die mechanische Belastung des Materials während der Expansion des Implantats hat Einfluss auf den Verlauf des Korrosionsprozesses und es ist davon auszugehen, dass die Spannungsrisskorrosion in den belasteten Bereichen verstärkt wird. Eine korrosionshemmende Schicht sollte diesen Umstand berücksichtigen. Weiterhin könnte eine harte korrosionshemmende Schicht während der Expansion des Stents abplatzen und eine Rissbildung in der Schicht bei Expansion des Implantats dürfte unvermeidbar sein. Schließlich sind die Dimensionen der filigranen metallischen Struktur zu beachten und es sollte nach Möglichkeit nur eine dünne, aber auch gleichmäßige korrosionshemmende Schicht erzeugt werden. Es hat sich nun gezeigt, dass das Aufbringen der erfindungsgemäßen Beschichtung ganz oder zumindest weitgehend diesen Anforderungen genügt.

Die Erfindung wird nachfolgend anhand eines Ausführungsbeispiels näher erläutert.

### Herstellung und Auswahl der Polymere:

Die Monomere Trimethylsilylmethacrylat TMSM (98%/Fa. Aldrich) und Methylmethacrylat MMA (99%/Fa. Aldrich) werden bei Bedarf vor der Polymerisation nach Perrin gereinigt (Perrin, Purification of Laboratory Chemicals). Azoisobutyronitril (AIBN) wird vor Gebrauch in Methanol umkristallisiert.

Verschiedene Verhältnisse der Monomere können wie folgt getestet werden. Es werden 1 molare Lösungen der Monomere in trockenem Toluol hergestellt und eine Stammlösung (1 molar) von AIBN in Toluol. Trockene Schraubdeckelreagenzgläser werden mit Stickstoff gespült und mit dem jeweiligen Anteil der Monomerlösung befüllt (siehe Tabelle). Die Polymerisation wird durch Zugabe der Initiatorlösung und Erhöhung der Temperatur gestartet. Nach ca. 15 Stunden wird die Polymerisation abgebrochen und das Polymer in Petrolether gefällt und abgetrennt. Nach zweimaligen Umfällen aus trockenem THF in Petrolether werden die Polymere analysiert.

| | V (TMSM) [mL] | V (MMA) [mL] | V (AIBN] [µL] |
|---|---|---|---|
| A | 1 | 9 | 100 |
| B | 2 | 8 | 100 |
| C | 3 | 7 | 100 |
| D | 4 | 6 | 100 |
| E | 5 | 5 | 100 |
| F | 6 | 4 | 100 |
| G | 7 | 3 | 100 |
| H | 8 | 2 | 100 |
| I | 9 | 1 | 100 |

Von den Polymeren sollten Folien mit einer Masse von jeweils etwa 100 mg hergestellt werden. Diese werden bei 37°C in künstlichem Plasma ausgelagert. Zum Beispiel nach 1, 8, 16, 30 und 90 Tagen werden die Folien gespült und getrocknet und der Massenverlust wird bestimmt.

### Beschichtung:

Je 10 Stents aus der kommerziell erhältlichen Magnesiumlegierung WE43 (Bezeichnung nach ASTM), die einen Seltenerdmetallanteil von etwa 3 Gew.% ohne Yttrium und einem Yttriumanteil von etwa 4 Gew.% aufweist, werden mit den Polymeren A bis I beschichtet. Die Beschichtungsmasse sollte 400 µg pro Stent betragen. Die Schichtdicke sollte ca. 4 µg betragen.

### Degradationsuntersuchungen:

Die beschichteten Stents sollten in künstlichem Plasma für 14 Tage ausgelagert werden. Danach kann die prozentuale Degradation der Stents beurteilt werden.

## Patentansprüche

1. Implantat mit einem Grundkörper, der ganz oder in Teilen aus einem biokorrodierbaren, metallischen Werkstoff besteht, und bei dem zumindest die aus dem biokorrodierbaren, metallischen Werkstoff bestehenden Teile des Grundkörpers vollständig oder teilweise mit einer Beschichtung bedeckt sind, die aus einem Polymer besteht oder dieses enthält, **dadurch gekennzeichnet, dass** das Polymer zu mindestens 90% der Gesamtzahl der vorhandenen Polymereinheiten aus Polymereinheiten der Formel (1) und Polymereinheiten der Formel (2) besteht 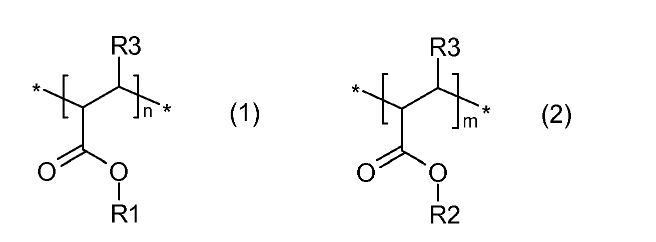 wobei R1 Alkyl, Hydroxyalkyl, Alkoxyalkyl oder Cycloalkyl ist;
R2 ein in künstlichem Plasma hydrolisierbarer Silylrest ist;
R3 Wasserstoff oder Methyl ist; und
n + m = 10 bis 20.000 ist, mit der Maßgabe, dass ein Verhältnis von n zu m in Bereich von 1:9 bis 9:1 liegt.

2. Implantat nach Anspruch 1, bei dem das Polymer durch Wahl der Substituenten R1 bis R3 sowie Festlegung von n und m eine Löslichkeit in künstlichem Plasma bei einer Temperatur von 37°C aufweist, die nach 24 Stunden in diesem Plasma geringer ist als 0.01 g/l.

3. Implantat nach Anspruch 1 oder 2, bei dem das Polymer durch Wahl der Substituenten R1 bis R3 sowie Festlegung von n und m eine Löslichkeit in künstlichem Plasma bei einer Temperatur von 37°C aufweist, die nach 30 Tagen in diesem Plasma im Bereich von 0.2 bis 0.5 g/l liegt.

4. Implantat nach einem der vorhergehenden Ansprüche, bei dem R1 ein unsubstituierter, hydroxy- oder C1-C10-alkoxysubstituierter C1-C10-Alkylrest oder ein C2-C10-Cycloalkylrest ist.

5. Implantat nach Anspruch 4, bei dem R1 Methyl, Ethyl, Propyl, 2-Methylethyl, Butyl, 2-Methylpropyl, 2,3-Dimethylethyl, Cyclohexyl, 2-Hydroxyethyl, 3-Hydroxypropyl oder 2-Methoxyethyl ist, bevorzugt Methyl, Ethyl, Propyl, oder Butyl und besonders bevorzugt Methyl ist.

6. Implantat nach einem der vorhergehenden Ansprüche, bei dem R2 Trimethylsilyl, Triethylsilyl, Tripropylsilyl oder Tributylsilyl ist und bevorzugt Trimethylsilyl oder Triethylsilyl ist.

7. Implantat nach einem der vorhergehenden Ansprüche, bei dem ein Verhältnis von n zu m im Bereich von 1:2,33 bis 1,5:1 liegt.

8. Implantat nach einem der vorhergehenden Ansprüche, bei dem die Polymereinheit der Formel (1) Methylmethacrylat oder Methylacrylat und die Polymereinheit der Formel (2) Trimethylsilylmethacrylat ist.

9. Implantat nach einem der vorhergehenden Ansprüche, bei dem ein mittleres Molekulargewicht der Polymere im Bereich von 1.000 bis 1.000.000 g/mol liegt.

10. Implantat nach Anspruch 9, bei dem das mittlere Molekulargewicht der Polymere im Bereich von 5.000 bis 500.000 g/mol liegt.

11. Implantat nach Anspruch 10, bei dem das mittlere Molekulargewicht der Polymere im Bereich von 40.000 bis 200.000 g/mol liegt.

12. Implantat nach einem der vorhergehenden Ansprüche, bei dem der biokorrodierbare metallische Werkstoff Reineisen oder eine biokorrodierbare Legierung ausgewählt aus der Gruppe Magnesium, Eisen, Zink, Molybdän und Wolfram ist.

13. Implantat nach Anspruch 12, bei dem der biokorrodierbare metallische Werkstoff eine Magnesiumlegierung ist.

14. Implantat nach einem der vorhergehenden Ansprüche, bei dem das Implantat ein Stent ist.

15. Implantat nach einem der vorhergehenden Ansprüche, bei dem das Polymer bis zu 10% Polymereinheiten aufweist, die durch radikalische Polymerisation eines von den Formeln (1) und (2) abweichenden Vinylmonomers entstehen.
